# EUROPEAN PATENT SPECIFICATION

(11) **EP 3 217 984 B1**
(45) Date of publication and mention of the grant of the patent: **12.09.2018**
(21) Application number: 15797633.3
(22) Date of filing: 13.11.2015
(51) Int. Cl.: A61K 31/522, A61P 43/00, A61P 21/00

(54) **CAFFEINE FOR THE TREATMENT OF MYOTONIC DYSTROPHY TYPE 1 AND TYPE 2**
KOFFEIN ZUR BEHANDLUNG VON MYOTONER DYSTROPHIE TYP 1 UND TYP 2
CAFÉINE POUR LE TRAITEMENT DE LA DYSTROPHIE MYOTONIQUE DE TYPE 1 ET DE TYPE 2

(30) Priority: 14.11.2014 EP 14382450
(43) Date of publication of application: 20.09.2017
(73) Proprietor: Universitat de València, 46010 Valencia (ES); Institut Químic de Sarrià CETS Fundació Privada, 08017 Barcelona (ES); Institut Univ. de Ciència i Tecnologia, S.A., 08100 Mollet del Vallès Barcelona (ES)
(72) Inventor: ARTERO ALLEPUZ, Ruben, E-46010 València (ES); CASTELLS BOLIART, Josep, E-08100 Mollet Del Valles (ES); BORRELL BILBAO, José Ignacio, E-08017 Barcelona (ES); LLAMUSI TROÍSI, Beatriz, E-46010 València (ES); BARGIELA SCHÖNBRUNN, Ariadna, E-46010 València (ES); KONIECZNY, Piotr, E-46010 València (ES); PASCUAL GILABERT, Marta, E-08100 Mollet Del Vallès (ES); TEIXIDÓ CLOSA, Jordi, E-08017 Barcelona (ES); ESTRADA TEJEDOR, Roger, E-08017 Barcelona (ES); LÓPEZ GONZÁLEZ, Alejandro, E-08017 Barcelona (ES)
(74) Representative: Ponti & Partners, S.L.P
(86) International application number: PCT/EP2015/076553
(87) International publication number: WO 2016/075288

(56) References cited:
- C CAMPBELL: "Congenital myotonic dystrophy", J NEUROL NEUROPHYSIOL, 23 January 2012 (2012-01-23), pages S7-001, XP055186364, ISSN: 2155-9562, DOI: 10.4172/2155-9562.S7-001 cited in the application
- KELLER C ET AL: "Congenital myotonic dystrophy requiring prolonged endotracheal and noninvasive assisted ventilation: not a uniformly fatal condition", PEDIATRICS, AMERICAN ACADEMY OF PEDIATRICS, EVANSTON, IL, US, vol. 101, no. 4, 1 April 1998 (1998-04-01) , pages 704-706, XP009184025, ISSN: 0031-4005
- QUEUDEVILLE M ET AL: "Congenital myotonic dystrophy with diaphragmatic paresis ; Kongenitale myotone Dystrophie mit Zwerchfellparese", MONATSSCHRIFT KINDERHEILKUNDE ; ORGAN DER DEUTSCHEN GESELLSCHAFTFÜR KINDERHEILKUNDE UND JUGENDMEDIZIN ORGAN DER ÖSTERREICHISCHENGESELLSCHAFT FÜR KINDERUND JUGENDMEDIZIN, SPRINGER, BERLIN, DE, vol. 160, no. 12, 21 June 2012 (2012-06-21), pages 1236-1238, XP035150576, ISSN: 1433-0474, DOI: 10.1007/S00112-012-2721-6
- SYR LEE ET AL: "Survival of a 30-week baby with congenital myotonic dystrophy initially ventilated for 55 days", JOURNAL OF PAEDIATRICS AND CHILD HEALTH, vol. 35, no. 3, 1 June 1999 (1999-06-01), pages 313-314, XP055186282, ISSN: 1034-4810, DOI: 10.1046/j.1440-1754.1999.00318.x
- M A RUTHERFORD ET AL: "Congenital myotonic dystrophy: respiratory function at birth determines survival.", ARCHIVES OF DISEASE IN CHILDHOOD, vol. 64, no. 2, 1 February 1989 (1989-02-01), pages 191-195, XP055186288, ISSN: 0003-9888, DOI: 10.1136/adc.64.2.191
- GRUENER R ET AL: "Caffeine-modulated acetylcholine sensitivity in denervated rat and diseased human muscle", LIFE SCIENCES, PERGAMON PRESS, OXFORD, GB, vol. 17, no. 10, 15 November 1975 (1975-11-15), pages 1557-1565, XP025525591, ISSN: 0024-3205, DOI: 10.1016/0024-3205(75)90177-0 [retrieved on 1975-11-15]

## Description

### Field of invention

The present invention relates to the field of rare diseases, in particular the invention relates to myotonic dystrophy. More particularly, the present invention relates to caffeine and compositions comprising thereof for use in the treatment of myotonic dystrophy type 1 and type 2.

### Background of the invention

Myotonic Dystrophy (DM) is the most common form of muscular dystrophy in adults and includes two clinically similar diseases although originating from distinct genetic mutations. DM1 (Steinert's disease) corresponds to the majority of the cases of DM and stems from an expansion of the CTG trinucleotide repeat (typically more than 50 units) in the 3 '-untranslated region (UTR) of the *DMPK* gene whereas DM2 originates from big expansions of the CCTG tetranucleotide in the first intron of the *CNBP* gene. Both are rare diseases, with a global combined prevalence estimated at 12.5 per 100000, although it has been suggested that it could be three times higher (Suominen, T. et al. Population frequency of myotonic dystrophy: higher than expected frequency of myotonic dystrophy type 2 (DM2) mutation in Finland. Eur J Hum Genet. 19(7):776-82, 2011).

Disease severity and age of onset are critically linked to expansion size. Whereas in classic adult onset the approximate number of repeats is 50 to <500, adolescent onset is 500 to <1000, childhood onset is 1000 to <1500, and congenital onset is equal or superior to 1500. Due to these differences, currently, there is not clear if the pathogenesis of congenital DM1 (CDM) is similar to adult DM1. In fact, unknown factors account for the unique features of CDM as there is no animal model of DM1 that produces a typical CDM phenotype (Campbell, et al. Congenital Myotonic Dystrophy J. Neurol. Neurophysiol. S7-001, XP055186364, 2012).

Cells carrying few CTG repeats show a functional equilibrium between two antagonistic splicing regulators: muscleblind-like 1 (MBNL1) and CUGBP/Elav-like family member 1 (CELF1). The balance between MBNL1 and CELF1 controls the establishment of adult splicing profiles for a subset of developmentally regulated transcripts. The CTG repeat expansion expressed in a DM1 cell forms an imperfect double-stranded stem loop structure that has two main pathogenic consequences: MBNL1 sequestration by RNA foci and protein kinase C (PKC)-mediated and AKT-mediated CELF1 hyperphosphorylation, stabilisation and subcellular redistribution. As a result of MBNL1 depletion and CELF1 upregulation, the balance between these two splicing regulators is disturbed and the alternative splicing of a series of developmentally regulated transcripts reverts to a foetal pattern. The abnormal expression of splicing isoforms in adult skeletal muscle, heart and brain is likely to contribute to the DM1 disease symptoms. Whereas MBNL1 seems critical in skeletal and cardiac phenotypes, sequestration of the MBNL2 paralog has been suggested to originate brain symptoms and MBNL3 may inhibit muscle differentiation. In addition to defective alternative splicing regulation, protein translation deficits, Repeat-Associated non-ATG translation (RAN translation) and bidirectional transcription of the *DMPK* gene, altered expression of microRNAs, gene transcription deficiencies, and RNA interference, among others, have been shown to contribute to molecular alterations typical of DM in different cell and animal models of the disease.

Under expression of MBNL or over expression of CELF1, or a combination of both situations as exists in human DM1 manifests in a multisystem illness due to modifications of RNA splicing.

DM1 is a truly multisystem disorder, primarily affecting skeletal muscles (myotonia, muscle weakness and degeneration), the heart and the central nervous system (CNS) (Gomes-Pereira, M. et al. Myotonic dystrophy mouse models: towards rational therapy development. Trends Mol Med. 17(9):506-17 (2011)). The great variability of DM1 symptoms and age of onset results in three main clinical forms of the disease: late-onset, classical adult onset and congenital DM1. DM1 can occur in patients of any age whereas DM2 typically presents in adults. Both DM1 and DM2 patients can also be considered as a person who carries the expanded CTG or CCTG mutation, respectively.

Myotonia (delayed muscle relaxation after initial contraction) and progressive wasting of distal muscles are prominent features of DM1 in skeletal muscle. The more severe congenital form of DM1 is characterized by general muscle hypotonia and respiratory distress at birth, as well as delayed motor development.

A large portion of patients suffer from cardiac conduction blocks, detected by electrocardiogram (ECG), and cardiac histological abnormalities. Progressive cardiopathy can result in complete atrioventricular block or ventricular arrhythmias and subsequent sudden death in almost 30% of DM1 patients. Particularly, problems related to the cardiopulmonary system accounts for 70% deaths due to DM1.

CNS manifestations are highly debilitating and support the view that DM1 is also a brain disorder. DM1 neuropsychological dysfunction is accompanied by histological abnormalities, as well as brain structural changes and altered metabolism.

The impact of DM1 further affects a variety of tissues and results in presenile cataracts, abnormal glucose tolerance and hyperinsulinism, gastrointestinal dysfunction and testicular atrophy. See Gomes-Pereira et al. Trends in Molecular Medicine, 2011, 17(9), 506-516

According to the Myotonic Dystrophy Foundation (http://www.myotonic.org), the symptomatology of the myotonic dystrophy can be expressed by the following events
1. Skeletal muscle: myotonia, progressive wasting and weakness, pain, general hypotonia in congenital DM1
2. Heart: cardiac condition defects, prolonged PR intervals, first degree atrioventricular block, arrhythmias
3. Central nervous system: hypersomnolence, cognitive impairment, executive dysfunction, visual-spacial memory deficits, neuropsychological changes, mental retardation in congenital DM1
4. Smooth muscle: gastrointestinal complications, swallowing issues, abdominal pain, abnormal motility, malabsorption, constipation/diarrhea, anal incontinence
5. Respiratory system: breathing problems in newborns, frequent lung infections, aspiration of food or fluids into airways, inability to breathe in enough oxygen, sleep apnea
6. Hormonal system: hyperinsulinism (diabetes), male prefrontal balding
7. Immune system: hypogammaglobulinemia.
8. Vision: premature subcapsular iridescent and muticoloured cataracts, damage to the retina, drooping eyelids (ptosis)
9. Reproductive system: testicular atrophy, small testes, reduced fertility, low sperm count, low testosterone, higher risk of miscarriage and stillbirth, early menopause, problems with pregnancy and delivery, newborn complications, more severe with each generation ("anticipation")
10. Skin: Higher risk of benign skin tumor (pilomatrixoma)

Current therapeutic strategies are based on candidate drugs that bind to CUG repeats, thereby releasing MBNL proteins to regulate splicing of its pre-mRNA targets. An increase in free MBNL1 protein has been shown to reduce the severity of symptoms in animal models of DM1, while in contrast, a decrease of free MBNL1 protein is observed with longer expansions, which are correlated with more severe disease. The pathogenic role of the MBNL1 gene is further substantiated by the fact that MBNL1 gene variants modify DM1 severity (Vincent Huin et al. MBNL1 gene variants as modifiers of disease severity in myotonic dystrophy type 1. J Neurol (2013) 260:998-1003). Similarly, MBNL proteins have been shown to get sequestered in DM2, thus, strategies aimed at increasing steady state levels of these proteins have the potential to become treatments for DM2. Then, the consensus strategy to reverse splicing abnormalities observed in DM1 is based in a reduction in nuclear foci and the concomitant reduction in nuclear sequestration of MBNL proteins. Warf and colleagues (Warf, M., Nakamori, M., Matthys, C., Thornton, C. and Berglund, J. (2009) Pentamidine reverses the splicing defects associated with myotonic dystrophy. Proceedings of the National Academy of Sciences of the United States of America, 106, 18551-18556. C. Thornton, C. and Berglund, J. (2009) Pentamidine reverses the splicing defects associated with myotonic dystrophy. Proceedings of the National Academy of Sciences of the United States of America, 106, 18551-18556. Coonrod, L., et al. (2013) Reducing levels of toxic RNA with small molecules. ACS Chem Biol. 8(11):2528-37) demonstrated that pentamidine facilitates a partial rescue of DM1 pathology.

Since then, some other therapies have been disclosed, such as antisense oligonucleotide compounds (see, EP 2 560 001A2, US 2011/0269665 A1), pentamidine analogues (see, A., Haley, M., Thornton, C. et al. (2013) Reducing levels of toxic RNA with small molecules. ACS chemical biology, 8, 2528-2537; Parkesh, R., Childs-Disney, J., Nakamori, M., Kumar, A., Wang, E., Wang, T., Hoskins, J., Tran, T., Housman, D., Thornton, C. et al. (2012) Design of a Bioactive Small Molecule That Targets the Myotonic Dystrophy Type 1 RNA via an RNA Motif-Ligand Database and Chemical Similarity Searching. Journal of the American Chemical Society, 134, 4731-4742), peptides (see, EP 2 554 180 A1, Gareiss, P., Sobczak, K., McNaughton, B., Palde, P., Thornton, C. and Miller, B. (2008) Dynamic combinatorial selection of molecules capable of inhibiting the (CUG) repeat RNA-MBNL1 interaction in vitro: discovery of lead compounds targeting myotonic dystrophy (DM1). Journal of the American Chemical Society, 130, 16254-16261; Garcia-Lopez, A., Llamusi, B., Orzaez, M., Perez-Paya, E. and Artero, R. (2011) In vivo discovery of a peptide that prevents CUG-RNA hairpin formation and reverses RNA toxicity in myotonic dystrophy models. Proceedings of the National Academy of Sciences of the United States of America, 108, 11866-11871), or chemical drug candidates (see, US 8.754.084 B2, EP 2 742 974 A1, US 8.741.572 B1, US 2014/0051709 A1, Jahromi, A.H., Nguyen, L., Fu, Y., Miller, K.A., Baranger, A.M. and Zimmerman, S.C. (2013) A novel CUG(exp). MBNL1 inhibitor with therapeutic potential for myotonic dystrophy type 1. ACS chemical biology, 8, 1037-1043; Wong, C.H., Nguyen, L., Peh, J., Luu, L.M., Sanchez, J.S., Richardson, S.L., Tuccinardi, T.,Tsoi, H., Chan, W.Y., Chan, H.Y. et al. (2014) Targeting Toxic RNAs that Cause Myotonic Dystrophy Type 1 (DM1) with a Bisamidinium Inhibitor. Journal of the American Chemical Society, 136, 6355-6361; Childs-Disney, J., Stepniak-Konieczna, E., Tran, T., Yildirim, I., Park, H., Chen, C., Hoskins, J., Southall, N., Marugan, J., Patnaik, S. et al. (2013) Induction and reversal of myotonic dystrophy type 1 pre-mRNA splicing defects by small molecules. Nature communications, 4, 2044; Hoskins, J.W., Ofori, L.O., Chen, C.Z., Kumar, A., Sobczak, K., Nakamori, M., Southall, N., Patnaik, S., Marugan, J.J., Zheng, W. et al. (2014) Lomofungin and dilomofungin: inhibitors of MBNL1-CUG RNA binding with distinct cellular effects. Nucleic acids research, 42, 6591-6602).

The generic cardiovascular drug mexiletine, initially developed to treat heart rhythm abnormalities, has been reported to hold some potential for treating muscle stiffness and other symptoms of non-dystrophic myotonias (NDMs). Mexiletine-induced sodium channel blockade reduced myotonia in small studies (Stantland, JM, et al. Mexiletine for symptoms and signs of myotonia in nondystrophic myotonia: a randomized controlled trial, JAMA. 2012, 308(13), 1357-1365).

Queudeville M et al. "Kongenitale myotone Dystrophie mit Zwerchfellparese", Monatsschrift Kinderheilkunde, 2012, vol. 160, No. 12, pp. 1236-1238, discloses theophylline as being able to improve the diaphragm contractility in child suffering from myotonic dystrophy (MD).

Syr Lee et al. "Survival of a 30-week baby with congenital myotonic dystrophy initially ventilated for 55 days", Journal of Paediatrics and Child Health, 1999, Vol. 35, No. 3, pp. 313-314, discloses the use of theophylline for enhancing the breathing effort of a child suffering from MD.

M.A. Rutherford et al. "Congenital myotonic dystrophy: respiratory function at birth determines survival", Archives of Disease in Childhood, 1989, Vol. 64, No. 2, pp. 191-195, discloses the use of aminophylline for enhancing the breathing effort of a child suffering from MD.

Keller C et al. "Congenital myotonic dystrophy requiring prolonged endotracheal and noninvasive assisted ventilation: not a uniformly fatal condition", Pediatrics, 1998, Vol. 101, No. 4, pp. 704-706, discloses caffeine as being able to enhance the breathing effort of a child suffering from MD.

Therefore, although DM1 was first described in 1909, there is presently no cure or specific treatment for myotonic dystrophy. All the treatments applied are palliative and contribute to control the development of a subset of the overall large group of sympthoms, and the clinical focus is on managing the complications of the disease, but in no case for treating the disease in a definitive manner.

Therefore, there is an existing need for compounds and compositions which can treat myotonic dystrophy, in particular myotonic dystrophy type 1 and type 2, in a more effective way.

The present inventors have surprisingly found that caffeine is a serious candidate for definitively treating DM, because its mechanism of action acts over the genetic etiology of the disease itself, in opposition to previous reported compounds that behave acting on a particular target of the symptomatology of the main related complications.

Caffeine is the best-known member of a family of drugs known as methylxanthines. Methylxantines occur naturally in a number of species of plants belonging to 28 genera and over 17 families, but their most common sources are coffee, tea, and chocolate. Caffeine is typically added to soft drinks and is an ingredient in many over-the-counter painkillers, cold remedies, headache remedies, stimulant pills, and, energy drinks. Caffeine acts by stimulating central nervous system, dieresis, cardiovascular and metabolic effects, bronchial relaxation and increased secretion of gastric acids. Recently, caffeine has been reported as having positive effects in the reduction of Parkinson's and Alzheimer's diseases (Drugs and Behavior: An Introduction to Behavioral Pharmacology, 7th Ed. William A. McKim, Stephanie D. Hancock, Peachpit Press (2013); Franco, R. et al. Health benefits of Methylxanthines in Cacao and Chocolate, Nutrients, 2013, 5, 4159-4173).

In spite of the large group of above mentioned caffeine applications, to our knowledge there is no prior art on the use of caffeine for the purpose claimed herein.

WO03/020977 relates to a method for identifying a substance capable of modulating the number of nucleotide repeats in a cell displaying expanded nucleotide repeat instability. This expansion of unstable nucleotide sequences is a primary genetic defect associated with DM1. This document states that caffeine is not able to bind to CUG repeats and increases the number of foci and consequently it is not a suitable candidate for drugs for treating myotonic dystrophy type 1 (DM1). Gomes-Pereira (Gomes-Pereira et al. 2004, Nuc. Acids Research, 2004, 32 (9), 2865-2872) discloses a study for identifying products that can reduce the rates of CAG-CTG repeat sequences, and that suppresion of somatic expansion would be expected to be therapeutic, or even curative in case of complete reversion. This document states that high doses of caffeine, close to 2 mM, increased the rate of expansion by *ca* 63%. Accordingly, the state of the art clearly discourages using caffeine as a candidate for an anti-DM therapeutic strategy.

Moreover, use of caffeine-free products are even recommended for DM patients (University of Maryland, Medical Center; https://umm.edu/health/medical/altmed/condition/muscular-dystrophy).

Nevertheless, the present inventors have focused the research on the grounds that overexpressing Muscleblind-like proteins, particularly MBNL1, has the potential of being therapeutic for this disease (see, Kanadia RN et al. Proc. Natl. Acad. Sci. USA 2006 Aug 1; 103 (31): 11748-53 and Chamberlain, C.M. et al. Human Molecular Genetics, 2012, 21(21), 4645-4654). The inventors have surprisingly found that, unlike any other xanthine derivative compounds, caffeine is able to increase the transcription of the human *MBNL1* promoter under the control of a muscle-specific *Drosophila muscleblind* (*mbl*) enhancer in transgenic flies, increasing thereby the expression of a enhanced Green Fluorescence Protein (eGFP) reporter. Importantly, caffeine was also able to increase the amount of free MBNL1 in human DM1 myoblasts.

An increase in free MBNL1 protein has been shown to reduce the severity of symptoms in animal models of DM1, while in contrast, a decrease of free MBNL1 protein is observed with longer expansions, which are correlated with more severe disease. The pathogenic role of the *MBNL1* gene is further substantiated by the fact that *MBNL1* gene variants modify DM1 severity (Vincent Huin et al. MBNL1 gene variants as modifiers of disease severity in myotonic dystrophy type 1. J Neurol (2013) 260:998-1003). Similarly, MBNL proteins have been shown to get sequestered in DM2, thus, strategies aimed at increasing steady state levels of these proteins have the potential to become treatments for DM2.

Caffeine, and xanthines in general, are known to act mainly as adenosine receptor blockers, commonly used for their effects as mild stimulants and as bronchodilators, notably in the treatment of asthma symptoms. In contrast to other more potent stimulants like sympathomimetic amines, they mainly act to oppose the actions of the sleepiness-inducing adenosine, and increase alertness in the central nervous system. They also stimulate the respiratory centre, and are used for treatment of infantile apnea and the apnea of prematurity (AOP). AOP is a common problem affecting premature infants, likely secondary to an immature respiratory system. Methylxanthine therapy is a mainstay of treatment of central apnea by stimulating the central nervous system and respiratory muscle function. The most common cause of apnea specially in newborns is prematurity, to whom the most common drugs used to treat apnea are the methylxanthines (Henderson-Smart et al. Prophylactic methylxanthine for prevention of apnoea in preterm infants. Cochrane Database Syst Rev. 2010 Dec 8;(12):CD000432. doi: 10.1002/14651858.CD000432.pub2); caffeine (Buk et al, Caffeine Citrate for the Treatment of Apnea of Prematurity, Pediatr Pharm. 2008;14(6)), theophylline (Scanlon et al. Caffeine or theophylline for neonatal apnoea? Arch Dis Child. 1992 Apr; 67(4 Spec No): 425-428) and aminophylline (Larsen et al. Aminophylline versus caffeine citrate for apnea and bradycardia prophylaxis in premature neonates. Acta Paediatr. 1995 Apr;84(4):360-4).

Methylated xanthines (methylxanthines), include caffeine, aminophylline, IBMX, paraxanthine, pentoxifylline, theobromine, and theophylline.

Therefore, according to the surprising finding by the present inventors, caffeine is considered a serious candidate for treating myotonic dystrophy type 1 (DM1) and myotonic dystrophy type 2 (DM2).

### Summary of the invention

A first aspect of the present invention relates to caffeine for use in reducing the severity of symptoms of myotonic dystrophy type 1 or type 2 in a subject suffering from myotonic dystrophy type 1 or type 2 by increasing the amount of free MBNL protein, said symptoms including those affecting the heart, central nervous system, smooth muscle, hormonal system, immune system, vision, reproductive system and skin.

A second aspect of the present invention relates to compositions comprising caffeine for use in reducing the severity of symptoms of myotonic dystrophy type 1 or type 2 in a subject suffering from myotonic dystrophy type 1 or type 2 by increasing the amount of free MBNL protein, said symptoms including those affecting the heart, central nervous system, smooth muscle, hormonal system, immune system, vision, reproductive system and skin.

### Brief description of the drawings

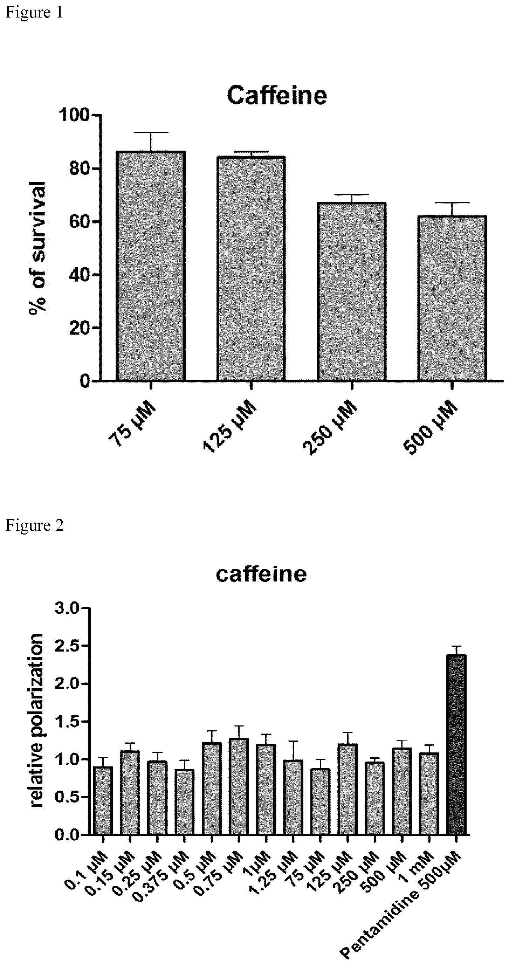

Figure 1 represents the percentage of survival of normal fibroblasts exposed to increasing concentrations of caffeine in the cell media. Even at the highest concentration the percentage of living cells was greater than 50%, thus caffeine is well tolerated by human fibroblasts. Data comes from three independent experiments. Error bars are s.e.m.
Figure 2 shows that caffeine does not bind CUG repeat RNA in fluorescence polarization assays. Bar graph shows polarization measurements relative to control alone from three independent experiments with four technical replicates each. Pentamidine was used as positive control. Error bars are s.e.m

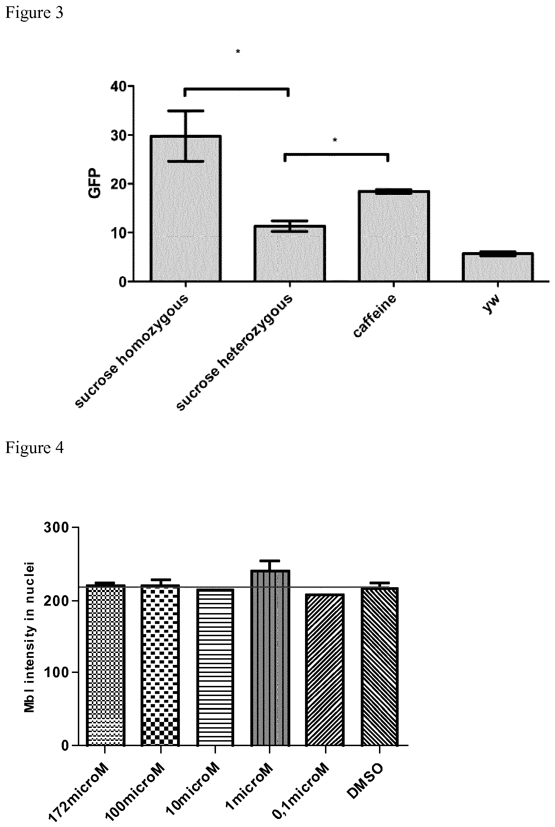

Figure 3 represents the quantification of eGFP expression from *yw* (negative control), homozygous (control of response to gene dosage) and heterozygous flies carrying the *ME:hsaP1-eGFP* construct, normalized to total amount of protein, taking caffeine or sucrose (dilutant). * indicates P<0.05. Data was generated from three biological replicates in arbitrary units of fluorescence. Error bars are s.e.m
Figure 4 represents the quantification of Mbl expression in DM1 cells. Bar graphs show the mean ± SEM of Mbl staining intensity in nuclei of DM1 myoblasts that had been treated with different concentrations of caffeine (from 0.1 microM to 172 microM) or solvent (DMSO) during one day. Horizontal red line marks the reference value of control cells treated only with DMSO.

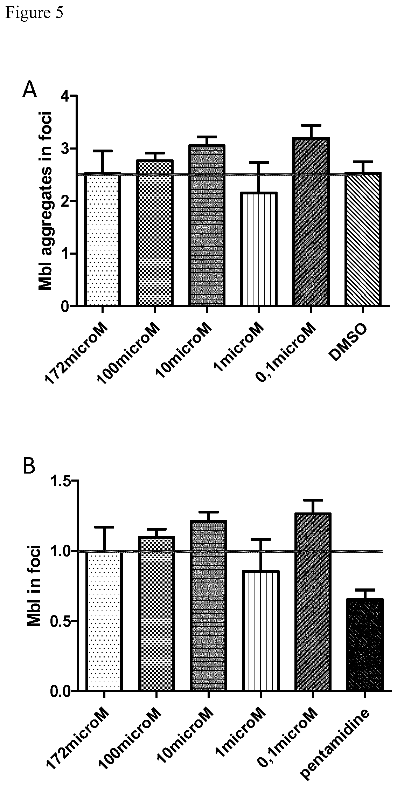

Figure 5 represents the quantification of Mbl expression in DM1 cells. Bar graphs show the mean ± SEM of number of Mbl aggregates in ribonuclear foci of DM1 myoblasts that had been treated with different concentrations of caffeine (from 0.1 microM to 172 microM) or solvent (DMSO) during one day (A). Horizontal red line marks the reference value of control cells treated only with DMSO. When pentamidine is used as positive control, the increase in Mbl aggregates in ribonuclear foci is obtained at all concentrations of caffeine (from 0.1 microM to 172 microM) (B).

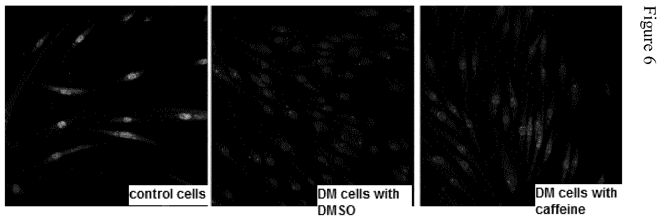

Figure 6 represents confocal microscope images of normal and DM1 myoblasts showing expression of the MBNL1 protein (green channel, cell nuclei are counterstained with DAPI in the blue channel). In normal myoblasts MBNL1 is detected preferentially in the cell nucleus, although it is also found in the cytoplasm. In DM1 myoblasts incubated with DMSO, as control, MBNL1 is characteristically sequestered in ribonuclear foci. Addition of 125 µM caffeine to the cell medium for two days increased the amount of MBNL1 that could be detected both in the cytoplasm and in the ribonuclear foci.

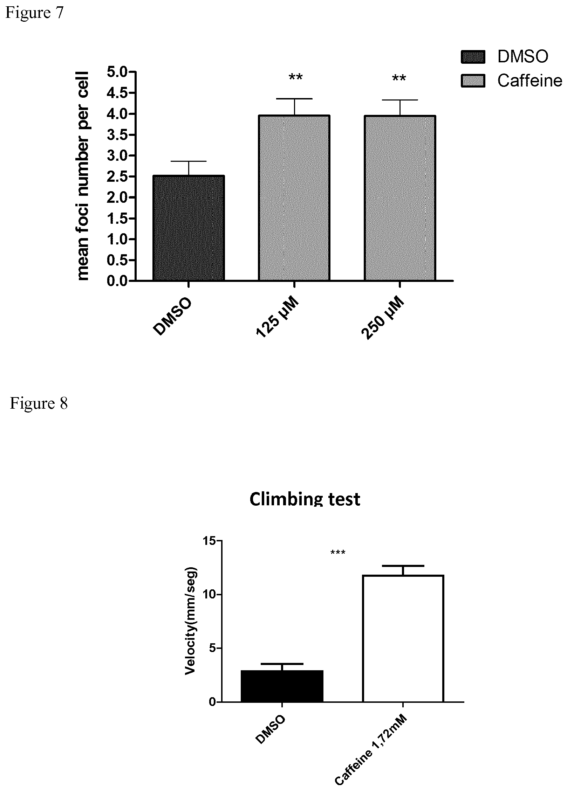

Figure 7 shows mean number of ribonuclear foci per cell in DM1 fibroblasts exposed to the indicated concentrations of caffeine in the culture media. Results show that caffeine increased the number of foci per cell. ** indicates p-value < 0.001. Data comes from three independent experiments with three technical replicates each. DMSO bar shows reference value for DM1 fibroblasts exposed to dilutant only. Error bars are s.e.m.
Figure 8 represents the functional assays to assess the effect of caffeine on locomotor performance in DM1 model flies. Bars show mean ± se of DM1 flies fed with standard nutritive media with DMSO (black) or caffeine to a final concentration of 1.72 mM (white). The climbing velocity is significantly improved in model flies fed with caffeine in comparison to model flies fed with the solvent DMSO.

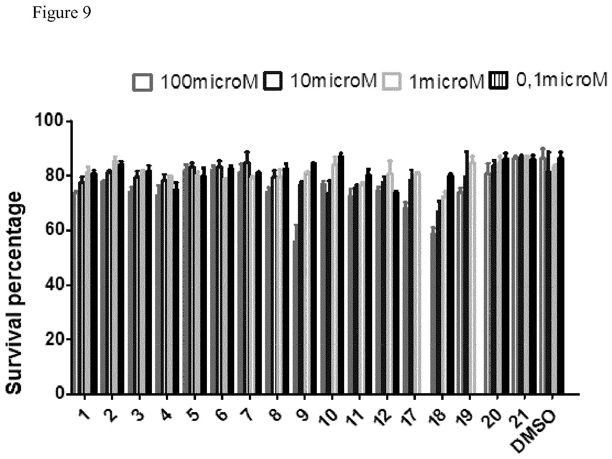

Figure 9 represents the percentage of survival of DM1 fibroblasts after administration of different concentrations of compounds in the media. The values of survival are normalized to non-treated DM1 fibroblasts which have 100% of survival. In all cases, the compounds were dissolved in DMSO, then the cells treated only with DMSO are considered the most appropriate controls for comparison. Bar graph shows means+s.e.m. from three independent experiments.

### Detailed description of the invention

The present invention relates to a compound which is caffeine 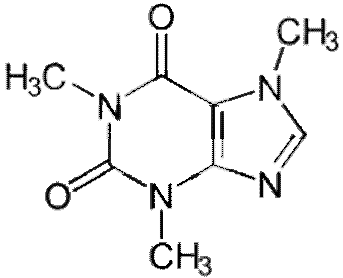 for use in reducing the severity of symptoms of myotonic dystrophy type 1 or type 2 in a subject suffering from myotonic dystrophy type 1 or type 2 by increasing the amount of free MBNL protein, said symptoms including those affecting the heart, central nervous system, smooth muscle, hormonal system, immune system, vision, reproductive system and skin.

In a particular embodiment, said compound is in the form of a tautomer, solvate, hydrate or a pharmaceutically acceptable salt thereof.

As used in the foregoing and hereinafter, the following definitions apply unless otherwise noted.

The present invention is also intended to include all isotopes of atoms occurring on the present compounds. Isotopes include those atoms having the same atomic number but different mass numbers. By way of general example and without limitation, isotopes of hydrogen include tritium and deuterium. Isotopes of carbon include C-13 and C-14.

The term "tautomer" or "tautomeric form" refers to structural isomer of different energies which are interconvertible via a low energy barrier. For example, proton tautomers (also known as prototropic tautomers) include interconversion via migration of a proton, such as keto-enol and imine-enamine isomerizations. Valence tautomers include interconversions by reorganization of some of the bonding electrons.

The present disclosure also includes the prodrugs leading to caffeine.

The term "prodrug" as used throughout this text means the pharmacologically acceptable derivatives such as esters, amides, and phosphates, such that the resulting *in vivo* biotransformation product of the derivative is the active drug as defined herein (caffeine). The reference by Goodman and Gilman (The Pharmacological Basis of Therapeutics, 8th ed, McGraw-Hill, Int. Ed. 1992, "Biotransformation of Drugs", p 13-15) describing prodrugs generally is hereby incorporated. Prodrugs preferably have excellent aqueous solubility, increased bioavailability and are readily metabolized into the active inhibitors *in vivo.* Prodrugs of caffeine may be prepared by modifying functional groups present in the compound in such a way that the modifications are cleaved, either by routine manipulation or *in vivo,* to the parent compound.

For therapeutic use, salts of caffeine are those wherein the counter-ion is pharmaceutically acceptable. "Pharmaceutically acceptable" as used herein means that the extract, fraction thereof, or compound thereof or composition is suitable for administration to a subject to achieve the treatments described herein, without unduly deleterious side effects in light of the severity of the disease and necessity of the treatment. However, salts of acids and bases which are non-pharmaceutically acceptable may also find use, for example, in the preparation or purification of a pharmaceutically acceptable compound. All salts, whether pharmaceutically acceptable or not are included within the ambit of the present invention.

The pharmaceutically acceptable acid and base addition salts as mentioned hereinabove are meant to comprise the therapeutically active non-toxic acid and base addition salt forms which caffeine is able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic and the like acids.

Conversely said salt forms can be converted by treatment with an appropriate base into the free base form.

The present invention also relates to the compound for use according to the set of claims which is included in a composition.

In a particular embodiment of said composition, the compound caffeine is in the form of a tautomer, solvate, hydrate or a pharmaceutically acceptable salt thereof.

In another embodiment of said composition, the composition further comprises at least a xanthine or a derivative thereof, wherein said derivative is selected from the group consisting of theophylline, theobromine, aminophylline, xanthine, hypoxanthine, 1,7-dimethylxanthine, 3-isobutyl-1-methylxanthine, 3-methylxanthine, 3-ethyl-1-propylxanthine, 3-allyl-1-ethyl-8-hydroxyxanthine, 3,8-dimethyl-2-thioxanthine, 1-ethyl-3 -isobutylxanthine, 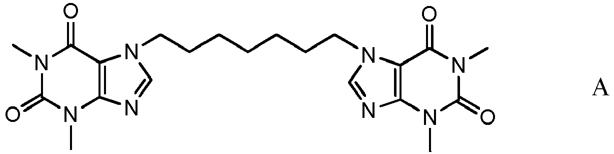 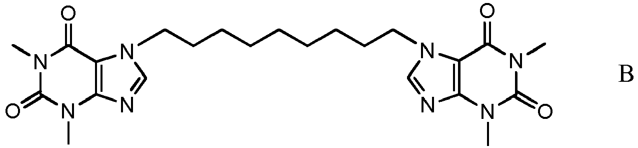 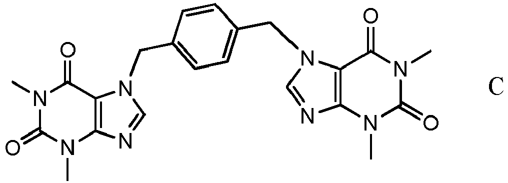 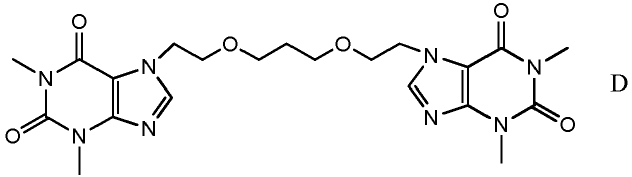 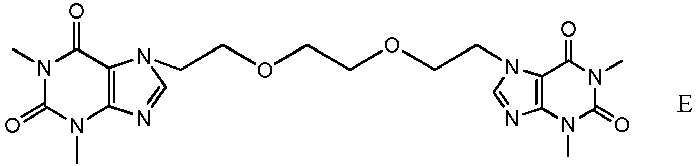 and

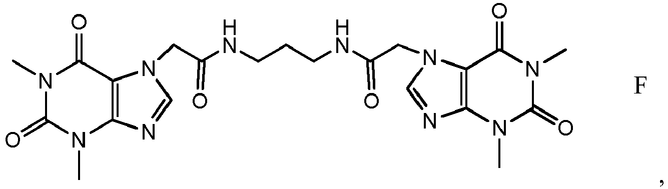

Preferably said derivative is theophylline, theobromine, aminophylline, hypoxantine, 1,7-dimethylxanthine, 3-isobutyl-1-methylxanthine, 3-ethyl-1-propylxanthine or 3-allyl-1-ethyl-8-hydroxyxanthine.

The present disclosure also relates to a method of treatment of myotonic dystrophy type 1 and type 2 in a subject comprising administering to said subject a therapeutically effective amount of the compound which is caffeine or a tautomer, solvate, hydrate or a pharmaceutically acceptable salt thereof. Preferably the subject is a human subject. In addition, the present disclosure also relates to a method of treatment of myotonic dystrophy type 1 or type 2 in a subject comprising administering to said subject a therapeutically effective amount of a composition comprising caffeine or a tautomer, solvate, hydrate or a pharmaceutically acceptable salt thereof. The phrase "therapeutically effective amount" means that amount of such a substance that produces some desired local or systemic effect at a reasonable benefit/risk ratio applicable to any treatment. The therapeutically effective amount of such substance will vary depending upon the subject and disease condition being treated, the weight and age of the subject, the severity of the disease condition, the manner of administration and the like, which can readily be determined by one of ordinary skill in the art. For example, certain compositions of the present disclosure may be administered in a sufficient amount to produce a reasonable benefit/risk ratio applicable to such treatment.

Lethal dose of caffeine in humans has been estimated at between 3 and 8 g (roughly 30 to 80 cups of coffee) (Drugs and Behavior: An Introduction to Behavioral Pharmacology, 7th Ed. William A. McKim, Stephanie D. Hancock, Peachpit Press (2013))

As used herein, a patient or subject who will suffer from myotonic dystrophy type 1 or 2 is defined as a person who carried any of the gene mutations disclosed in the background section, even though the disease has not developed.

In another preferred embodiment, the composition for use according to the present invention is formulated as a pharmaceutical composition, food, food ingredient or supplement, nutraceutical composition, additive for a natural product or is present in the extract of a natural product. Preferably, said composition is the form of a solid or liquid. More preferably, said composition is present in a dairy product, a beverage, a food supplement, a nutraceutical composition or cereals.

A composition of a "food" item or "food ingredient or supplement" as described above may in principle take any form suited for consumption by man or animal. In one embodiment the composition is in the form of a dry powder that can be suspended, dispersed, emulsified or dissolved in an aqueous liquid such as water, coffee, tea, milk, yogurt, stock or fruit juice and alcoholic drinks. To this end, the powder may be provided in unit-dosage form. In an alternative preferred embodiment the composition in the form of a dry powder is tabletted. To that end, a composition for a food supplement according to the invention may very suitably be provided with fillers, such as microcrystalline cellulose (MCC) and mannitol, binders such as hydroxypropylcellulose (HPC), and lubricants such as stearic acid or other excipients. A composition of a food item or food supplement as described above may also be provided in the form of a liquid preparation wherein the solids are suspended, dispersed or emulsified in an aqueous liquid. Such a composition may be admixed directly through a food item or may e.g. be extruded and processed to grains or other shapes. In an alternative embodiment a food item or food supplement may take the shape of a solid, semi-solid or liquid food item, such as cereals, a bread, a bar, a cookie, a sandwich or a beverage, or as a spread, sauce, butter, margarine, dairy product, and the like. Preferably, the composition is included in a dairy product, such as for instance a butter or margarine, custard, yogurt, cheese, spread, drink, or pudding or other dessert.

Nutraceuticals can be defined as natural products that are used to supplement the diet by increasing the total dietary intake of important nutrients. This definition includes nutritional supplements such as vitamins, minerals, herbal extracts, antioxidants, amino acids, and protein supplements. Nutraceutical products fit into the newly created product category of "Dietary Supplements" as established by the F.D.A. in the Dietary Supplement Act of 1994. This act specifically defined dietary supplements to include: vitamins, minerals, herbs or other botanicals, antioxidants, amino acids, or other dietary substances used to supplement the diet by increasing the total daily intake. A "nutraceutical composition" is defined herein as a food composition fortified with ingredients capable of producing health benefits. Such a composition in the context of the present invention may also be indicated as foods for special dietary use; medical foods; and dietary supplements. For example, the food item or supplement may help to prevent or reduce symptoms associated with an inflammatory condition such as allergies (e.g. hay fever) and the like. As with the pharmaceutical composition, the amount of active ingredient in the food or food additive will depend on several factors. The food product will generally comprise a concentration that is sufficient to provide a consumer with an effective amount of active ingredient upon consumption of a regular (e.g. daily) portion of the food product. It will be recognized by those skilled in the art that the optimal quantity and spacing of individual dosages for achieving the therapeutic effects of the pharmaceutical composition, food item or food supplement described herein may easily be determined by the skilled person.

As mentioned above, the compounds or compositions disclosed herein can also be present in natural products. For example, it is well known that caffeine is found in varying quantities in the seeds, leaves, and fruit of some plants. It is for example extracted from the seed of the coffee plant and the leaves of the tea bush, as well as from various foods and drinks containing products derived from the kola nut. Other sources include yerba maté, guarana berries, guayusa, and the yaupon holly.

In another preferred embodiment, the compound or composition disclosed herein is administered by oral, rectal, nasal, topical, vaginal, parenteral, transdermal, intraperitoneal, intrapulmonary and intranasal route.

In another preferred embodiment, the compound or composition disclosed herein is administered to a patient having a non-congenital form of DM.

Dose ranges of the pharmaceutical compositions can be adjusted as necessary for the treatment of individual patients and according to the specific condition treated. As it is well known in the art, single doses of caffeine up to 200 mg (about 3 mg/kg bw for a 70-kg adult) do not give rise to safety concerns (See EFSA Journal 2015;13(5):4102). Habitual caffeine consumption up to 400 mg per day does not give rise to safety concerns for non-pregnant adults. The Panel considers that caffeine intakes of no concern derived for acute caffeine consumption by adults (3 mg/kg bw per day) may serve as a basis to derive single doses of caffeine and daily caffeine intakes of no concern for these population subgroups.

Any of a number of suitable pharmaceutical formulations may be utilized as a vehicle for the administration of the compositions of the present invention and maybe a variety of administration routes are available. The particular mode selected will depend of course, upon the particular formulation selected, the severity of the disease, disorder, or condition being treated and the dosage required for therapeutic efficacy. The methods of this invention, generally speaking, may be practiced using any mode of administration that is medically acceptable, meaning any mode that produces effective levels of the active compounds without causing clinically unacceptable adverse effects. Such modes of administration include oral, rectal, topical, nasal, transdermal or parenteral routes and the like. Accordingly, the formulations of the invention include those suitable for oral, rectal, topical, buccal, sublingual, parenteral (e.g., subcutaneous, intramuscular, intradermal, inhalational or intravenous) and transdermal administration, although the most suitable route in any given case will depend on the nature and severity of the condition being treated and on the nature of the particular active product used.

Formulations suitable for oral administration may be presented in discrete units, such as capsules, cachets, lozenges, drops, or tablets, each containing a predetermined amount of the active compound; as a powder or granules; as a solution or a suspension in an aqueous or non-aqueous liquid; or as an oil-in-water or water-in-oil emulsion. Such formulations may be prepared by any suitable method of pharmacy which includes the step of bringing into association the active compound and a suitable carrier (which may contain one or more accessory ingredients as noted above).

In general, the formulations of the invention are prepared by uniformly and intimately admixing the active compound with a liquid or finely divided solid carrier, or both, and then, if necessary, shaping the resulting mixture. For example, a tablet may be prepared by compressing or molding a powder or granules containing the active compound, optionally with one or more accessory ingredients. Compressed tablets may be prepared by compressing, in a suitable machine, the compound in a free-flowing form, such as a powder or granules optionally mixed with a binder, lubricant, inert diluent, and/or surface active/dispersing agent(s). Molded tablets may be made by molding, in a suitable machine, the powdered compound moistened with an inert liquid binder.

Formulations of the present invention suitable for parenteral administration conveniently comprise sterile aqueous preparations of the active compound, which preparations are preferably isotonic with the blood of the intended recipient. These preparations may be administered by means of subcutaneous, intravenous, intramuscular, inhalational or intradermal injection. Such preparations may conveniently be prepared by admixing the compound with water or a glycine buffer and rendering the resulting solution sterile and isotonic with the blood.

Formulations of the inventive mixtures are particularly suitable for topical application to the skin and preferably take the form of an ointment, cream, lotion, paste, gel, spray, aerosol, or oil. Carriers which may be used include Vaseline, lanoline, polyethylene glycols, alcohols, transdermal enhancers, and combinations of two or more thereof.

Formulations suitable for transdermal administration may also be presented as medicated bandages or discrete patches adapted to remain in intimate contact with the epidermis of the recipient for a prolonged period of time. Formulations suitable for transdermal administration may also be delivered by iontophoresis (passage of a small electric current to "inject" electrically charged ions into the skin; also called electromotive drug administration (EMDA)) through the skin.

The present invention is now further illustrated by reference to the following examples.

### EXAMPLES

### Caffeine is non-toxic for human fibroblasts cells

Reference is made to figure 1.

### Materials and Methods

Fibroblast cells were grown in DMEM with 4.5g/L of glucose, 1% of penicillin and streptomycin (P/S) and 10% foetal bovine serum (FBS) (Sigma). Cells were aliquoted in 96-well plate with 1.0 x 10⁴ cells per well and incubated for 24 h. Caffeine (Sigma-Aldrich) was added to a final concentration of 75 µM, 125 µM, 250 µM and 500 µM in DMEM and cells were incubated for 24 h. To measure cell viability, MTS tetrazolium salt was added to each well and was incubated for 4 h at 37°C in a humidified chamber with 5% CO₂. The conversion of MTS into soluble formazan (accomplished by dehydrogenase enzymes from metabolically active cells) was measured by absorbance at 490 nm (CellTiter 96® Aqueous Non-Radioactive Cell Profileration Assay, Promega). Data were transformed to percentage of survival relative to cells not exposed to caffeine, which established 100% viability.

### Caffeine cannot bind CUG expansions in fluorescence polarization spectroscopy experiments

This was assessed in fluorescence polarization spectroscopy experiments in which fluorescent CUG RNA probe (carboxyfluorescein (6-FAM)-labeled) was incubated in increasing concentrations of caffeine. Whereas 6-FAM-CUG RNA molecules do not fluorescence in any particular polarization axis, binding to a molecule slows down the rotational movement of the molecule and increases polarization values.

**Results for caffeine:** caffeine did not increase polarization values of 6-FAM-CUG RNA even at concentrations twice those employed with the positive control pentamidine, which suggest that caffeine was unable to bind to the CUG repeats and therefore cannot prevent sequestration of MBNL proteins in ribonuclear foci.

See fig. 2.

### Materials and Methods

### Polarization fluorescence assays

Carboxyfluorescein-labeled CUG RNA (23 CUG repeats; 6-FAM-CUG₂₃) at 6 nM was incubated with caffeine at different concentrations in binding buffer (50 mM Tris-HCl pH 7.0, 250 mM NaCl, 50 µM ZnCl₂, 10% glycerol, 1 mM DTT) on ice for 20 min in the dark. Polarization was measured in a En Vision® Multilabel Reader using as excitation filter FP480 and as emission filter FP535.

### Caffeine increases free Muscleblind protein

### • Evidences from Drosophila:

We have previously characterized the transcriptional regulation of the *muscleblind* gene in *Drosophila* (Bargiela et al. Two enhancers control transcription of Drosophila muscleblind in the embryonic somatic musculature and in the central nervous system. 2014 Mar 25;9(3):e93125) and have identified a defined genomic regulatory region able to drive the expression of *muscleblind* in all somatic muscles ("ME" enhancer region) and a minimal promoter from the human *MBNL1* ortholog (*hsaP1*)*.* In particular, ME placed upstream of the endogenous *MBNL1* promoter and of the enhanced Green Fluorescence Protein (eGFP) reporter expressed eGFP throughout the *Drosophila* somatic musculature in transgenic flies (*ME:hsaP1-eGFP* flies).

Flies heterozygous for the *ME:hsaP1-eGFP* construct were used in a chemical screen searching for compounds capable of modifying the expression of the reporter and thus, potentially, transcription of the *muscleblind* locus. Caffeine was found to significantly increase the expression of the eGFP reporter. Therefore, it is inferred that caffeine has the ability to increase transcription of the endogenous *muscleblind* gene.

See fig. 3.

### Materials and Methods

*ME:hsaP1-eGFP* flies were crossed to *yw* to obtain heterozygous offspring. 2-day-old heterozygous flies were used for *in vivo* testing of compounds, serving *yw* and homozygous flies as controls of background and increased reporter expression, respectively. 25 mg/ml of caffeine (Sigma Aldrich) was dissolved in standard fly food (final concentration of the sucrose dilutant in food, 10%). Five flies per tube were used in each test and the experiment was carried out for 8 h at 25°C. Flies with the same genotype but taking sucrose 10% in standard food served as controls. Three biological replicates per group were performed. To measure eGFP, three flies were homogenized and eGFP was measured using a Tecan plate reader. Total amount of protein was quantified using the BCA protein assay kit (Pierce) and was used to normalize eGFP measurements.

### • Caffeine increases Mbl expression intensity in nuclei

This parameter measures the mean intensity of Mbl staining per pixel, only in pixels inside the nuclei, which are marked by DAPI. Increased Mbl intensity in nuclei denotes an increment in Mbl expression.

See fig. 4.

### • Caffeine increases Mbl aggregates ribonuclear foci

This parameter measure the number of ribonuclear foci containing Mbl. An increased number of Mbl aggregates in foci means increased Mbl expression. A decreased number of Mbl in foci could mean; (1) general decreased of Mbl expression or (2) decreased Mbl sequestration in foci, when it is accompanied by an increased Mbl intensity in nuclei.

See fig. 5 (A) and 5 (B).

### • Caffeine increases MBNL1 in human DM1 myoblasts

To investigate whether caffeine had an effect on MBNL1 expression levels in human cells we immunodetected MBNL1 in normal and control DM1 myoblasts, and in DM1 myoblasts grown in 125 and 250 µM caffeine. At both concentrations MBNL1 expression increased in the cytoplasm and in the cell nucleus compared to DM1 myoblasts exposed to DMSO alone (solvent of caffeine). The observed increase of MBNL1 expression could originate from a number of ways: release from ribonuclear foci, increase in transcription or enhanced protein stability. However, the number of MBNL1-containing foci in caffeine-treated cells was qualitatively higher than in DMSO-treated cells, which is not consistent with a model of release from CUG expansions. Thus, caffeine increases expression of MBNL1. Because the levels of MBNL1 in DM1 myoblasts are limiting, an increase in their levels, even if ribonuclear foci increase, is potentially therapeutic.

See fig. 6.

See fig. 7.

### Materials and Methods

### Cell culture conditions

Cell model of the disease (provided by the D. Furling's laboratory, Institute of Myologie, Paris) consisted of normal and DM1 (1300 CTG repeats) immortalized (hTERT) skin fibroblasts expressing conditional MyoD. Fibroblast cells were grown in Dulbecco's Modified Eagle Medium (DMEM) with 4.5 g/L of glucose, 1 % of penicillin and streptomycin (P/S) and 10% foetal bovine serum (FBS) (Sigma). Fibroblasts were transdifferentiated in myoblasts by inducing expression of MyoD. Cells were plated in muscle differentiation medium (MDM) made of DMEM 4.5 g/L glucose with 1% P/S, 2% horse serum, 1% apo-transferrin (10 mg/ml), 0,1 % insulin (10 mg/ml) and 0,02 % doxycyclin (10 mg/ml) for 48 h. For compound testing fibroblasts were aliquoted in 24-well plate with 3.5 x 10⁴ cells per well and were differentiated as before. Caffeine (Sigma-Aldrich) was added to a final concentration of 125 µM and 250 µM in MDM medium and cells were incubated for 48 h.

### Immunodetection of MBNL1

Cells were fixed in 4% paraformaldehyde (PFA) for 10 min at room temperature followed by several washes in PBS 1x. Cells were then permeabilized with 0.3 % Triton in PBS (PBT), blocked (PBT 1% donkey serum) for 30 min at room temperature, and incubated with primary antibody (mouse anti-MBNLl 1:500; Sigma) at 4°C overnight. After several washes with PBT cells were incubated for 45 min with biotin-conjugated secondary antibody (Sigma) at a 1:200 dilution. Cells were then incubated with ABC solution (ABC kit, VECTASTAIN) for 30 min at room temperature, followed by PBT washes and incubation with streptavidin-FITC (1:1000) for 20 min. Samples were finally mounted in Vectashield (Vector) with 2 µg/ml DAPI.

### Foci detection

*In situ* hybridization with CUG repeat RNA. Cells were fixed in 4% paraformaldehyde (PFA) for 10 min at room temperature followed by several washes in PBS 1x. Fixed cells were incubated in pre-hybridization buffer (SSC 2x, 30% deionized formamide) for 10 min at room temperature, hybridized with Cy3-(CAG)₇-Cy3 labelled probe diluted 1:100 in hybridization buffer (40% formamide, 2x SSC, 0.2% BSA, 10% dextran sulfate, 2 mM vanadyl complex, 10% tRNA (10 mg/ml), 10% herring sperm) for 2 h at 37°C, washed twice in pre-hybridization buffer for 15 min at 45°C, washed in PBS 1x for 15 min at room temperature and mounted in Vectashield (Vector) with 2 µg/ml DAPI. Images were taken using a Leica DM2500 fluorescence microscope and foci were manually counted from at least 50 cells per compound.

See fig. 7

### Caffeine improves leg muscles movement in Drosophila

*In vivo* functional study: Climbing assay

To assess climbing velocity, after emerging, the flies were transferred to tubes with 1.72 mM caffeine or the dissolvent DMSO 1% in standard nutritive media. Groups of ten 5-day-old males were transferred into vials of 1.5 cm in diameter and 25 cm in height, after a period of 24 hours without anesthesia. The height reached from the bottom of the vial by each fly in a period of 10 s was recorded with a camera. For each genotype, approximately 30 flies were tested. The results show the mean speed in mm/s. We used a t-test to assess the statistical difference between the two groups of flies, fed with or without compound.

Reference is made to figure 8.

Here we report the surprising finding that caffeine promotes higher levels of MBNL1 in human DM1 myoblasts (Fig. 4) and boosts expression of a reporter of the *Drosophila melanogaster* homologue gene in transgenic animals (Fig. 3). Furthermore, caffeine does not bing to CUG repeat RNA according to polarization spectroscopy assays (Fig. 2), increases the mean number of foci per myoblast in a cell model of the disease (Fig. 6) and increases Mbl both in nuclei (Fig. 4) and in ribonuclear foci (Fig. 5). Functional tests allow the assessment of muscle atrophy rescue achieved by a compound. Improve results in these tests require increase of muscle mass, strength and coordination. In the case of caffeine, at 1.72 mM this compound was able to rescue climbing (Fig. 8). Finally, caffeine is particularly well tolerated by human fibroblasts in cell culture conditions (Fig. 1).

Taken together, these data are consistent with caffeine being able to promote expression of MBNL1 in human cells, instead of releasing MBNL1 from CUG foci, as previously reported for other small molecules. Because there is ample evidence that MBNL1 activity is limiting in DM1 and DM2 diseases due to sequestration by CUG and CCUG repeat expansions, that strategies aimed at increasing the amount of free MBNL1 are therapeutic in cell and animal experimentation models, and that MBNL1 overexpression is well-tolerated in skeletal muscle and is compatible with life, caffeine is then proposed to be used in the treatment of DM1 and DM2 in patients.

### Compositions comprising caffeine and at least a xanthines or derivative thereof are non-toxic for human fibroblasts cells

To assess the toxicity of compounds in culture cells, different concentrations of compounds (from 0.1 to 100 microM) were added to standard media in DM1 fibroblasts and studied survival using the CellTiter 96 Aqueous Non-Radioactive cell proliferation assay protocol; colorimetric assay that determines viable cells.

As the percentage of survival was always above 50% in comparison to non-treated cells and above 75% in comparison to DMSO treated cells, all the concentrations were continued being used in the following assays of activity, as their toxicity was very low. Therefore, taken together caffeine and their compositions comprising at least a xanthine are non-toxic for human fibroblasts.

See fig. 1 and fig. 9

### Compositions comprising caffeine and at least a xanthine or derivative thereof increase MBNL1 in human DM1 myoblasts

To investigate whether compositions comprising caffeine and at least a xanthine had an effect on MBNL1 expression levels in human cells we immunodetected MBNL1 in normal and control DM1 myoblasts, and in DM1 myoblasts grown in a range of combinations comprising caffeine (see Table 1). MBNL1 expression increases in the cytoplasm and in the cell nucleus compared to DM1 myoblasts exposed to DMSO alone (used as solvent and negative control).

**Table 1**

| **Sample** | **Component 1** | **Component 2** | **Component 3** | **Relative ratio** |
|---|---|---|---|---|
| 1 | Caffeine | Theophylline | - | 2:1 |
| 2 | Caffeine | Theophylline | - | 1:1 |
| 3 | Caffeine | Theophylline | - | 1:2 |
| 4 | Caffeine | Theobromine | - | 2:1 |
| 5 | Caffeine | Theobromine | - | 1:1 |
| 6 | Caffeine | Theobromine | - | 1:2 |
| 7 | Caffeine | Aminophylline | - | 2:1 |
| 8 | Caffeine | Aminophylline | - | 1:1 |
| 9 | Caffeine | Aminophylline | - | 1:2 |
| 10 | Caffeine | Theophylline | Theobromine | 1:1:1 |
| 11 | Caffeine | Theophylline | Theobromine | 2:1:2 |
| 12 | Caffeine | Theophylline | Theobromine | 1:1:2 |
| 1 | Caffeine | 1,7-Dimethylxanthine | - | 1:1 |
| 3 | Caffeine | 1,7-Dimethylxanthine | - | 1:2 |
| 14 | Caffeine | 1,7-Dimethylxanthine | - | 2:1 |
| 15 | Caffeine | 3 -Isobutyl-1-methylxanthine | - | 1:1 |
| 16 | Caffeine | 3 -Isobutyl-1-methylxanthine | - | 1:2 |
| 18 | Caffeine | 3 -Isobutyl-1-methylxanthine | - | 2:1 |
| 19 | Caffeine | 3-Methylxanthine | - | 1:1 |
| 20 | Caffeine | 3-Methylxanthine | - | 1:2 |
| 21 | Caffeine | 3-Methylxanthine | - | 2:1 |
| 22 | Caffeine | 3 -Ethyl-1-propylxanthine | - | 1:1 |
| 23 | Caffeine | 3 -Ethyl-1-propylxanthine | - | 1:2 |
| 24 | Caffeine | 3 -Ethyl-1-propylxanthine | - | 2:1 |
| 25 | Caffeine | 1,7-Dimethylxanthine | 3-Isobutyl-1-methylxanthine | 1:1:1 |
| 26 | Caffeine | 1,7-Dimethylxanthine | 3-Isobutyl-1-methylxanthine | 2:1:2 |
| 27 | Caffeine | 1,7-Dimethylxanthine | 3-Isobutyl-1-methylxanthine | 1:1:2 |
| 28 | Caffeine | 3 -Isobutyl-1-methylxanthine | 3-Methylxanthine | 1:1:1 |
| 28 | Caffeine | 3 -Isobutyl-1-methylxanthine | 3-Methylxanthine | 2:1:2 |
| 30 | Caffeine | 3 -Isobutyl-1-methylxanthine | 3-Methylxanthine | 1:1:2 |
| 31 | Caffeine | 3 -Isobutyl-1-methylxanthine | 3-Ethyl-1-propylxanthine | 1:1:1 |
| 32 | Caffeine | 3 -Isobutyl-1-methylxanthine | 3-Ethyl-1-propylxanthine | 2:1:2 |
| 33 | Caffeine | 3 -Isobutyl-1-methylxanthine | 3-Ethyl-1-propylxanthine | 1:1:2 |

## Claims

1. A compound which is caffeine 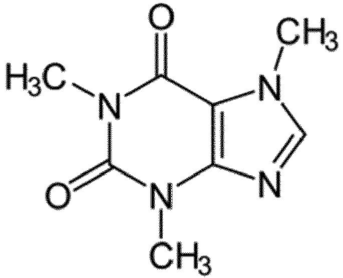 for use in reducing the severity of symptoms of myotonic dystrophy type 1 or type 2 in a subject suffering from myotonic dystrophy type 1 or type 2 by increasing the amount of free MBNL protein, said symptoms including those affecting the heart, central nervous system, smooth muscle, hormonal system, immune system, vision, reproductive system and skin.

2. The compound for use, according to claim 1, which is in the form of a tautomer, solvate, hydrate or a pharmaceutically acceptable salt thereof.

3. The compound for use, according to claim 1 or 2, which is included in a composition.

4. The compound for use, according to claim 3, wherein said composition further comprises at least a xanthine or a derivative thereof, wherein said derivative is selected from the group consisting of theophylline, theobromine, aminophylline, hypoxanthine, 1,7-dimethylxanthine, 3-isobutyl-1-methylxanthine, 3-methylxanthine, 3-ethyl-1-propylxanthine, 3-allyl-1-ethyl-8-hydroxyxanthine, 3,8-dimethyl-2-thioxanthine, 1-ethyl-3-isobutylxanthine, 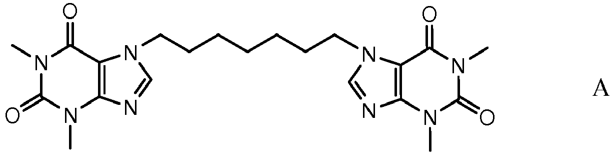 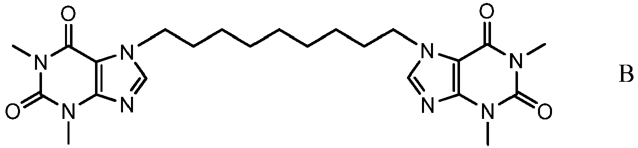 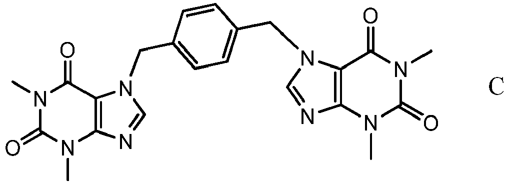 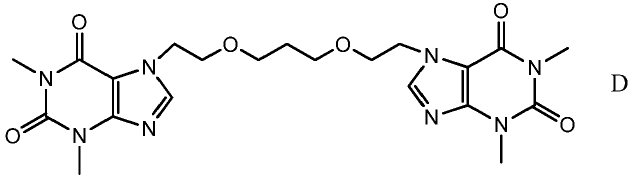 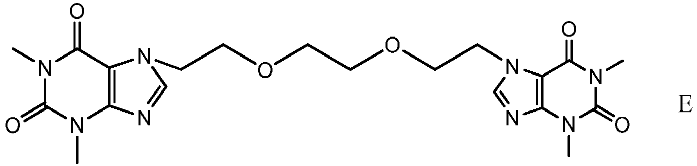 and

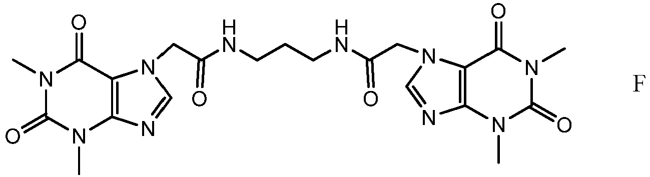

5. The compound for use, according to claim 4, wherein said derivative is theophylline, theobromine, aminophylline, hypoxantine, 1,7-dimethylxanthine, 3-isobutyl-1-methylxanthine, 3-ethyl-1-propylxanthine or 3-allyl-1-ethyl-8-hydroxyxanthine.

6. The compound for use, according to any of claims 3 to 5, wherein said composition is formulated as a pharmaceutical composition, food, food ingredient or supplement, nutraceutical composition, additive for a natural product or is present in the extract of a natural product.

7. The compound for use, according to any of claims 3 to 6, wherein said composition is in the form of a solid or liquid.

8. The compound for use, according to any of claims 3 to 7, wherein said composition is present in a diary product, a beverage or cereals.

9. The compound for use, according to any of the preceding claims, which is administered by oral, rectal, nasal, topical, vaginal, parenteral, transdermal, intraperitoneal, intrapulmonary and intranasal route.

## Patentansprüche

1. Eine Verbindung, die Koffein ist: 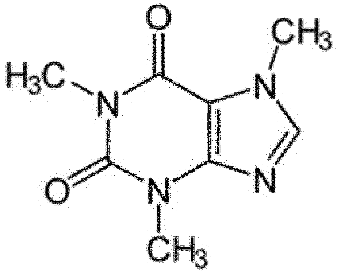 zur Verwendung beim Reduzieren der Schwere von Symptomen der myotonen Dystrophie Typ 1 oder Typ 2 bei einer Person, die an myotoner Dystrophie Typ 1 oder Typ 2 leidet, durch Erhöhen einer Menge eines freien MBNL-Proteins, wobei die Symptome diejenigen beinhalten, die Herz, zentrales Nervensystem, glatte Muskeln, Hormonsystem, Immunsystem, Sehvermögen, Reproduktionssystem und Haut betreffen.

2. Die Verbindung zur Verwendung gemäß Anspruch 1, die in Form eines Tautomers, Solvats, Hydrats oder eines pharmazeutisch annehmbaren Salzes davon vorliegt.

3. Die Verbindung zur Verwendung gemäß Anspruch 1 oder 2, die in einer Zusammensetzung enthalten ist.

4. Die Verbindung zur Verwendung gemäß Anspruch 3, wobei die Zusammensetzung ferner zumindest ein Xanthin oder ein Derivat davon umfasst, wobei das Derivat ausgewählt ist aus der Gruppe, die besteht aus Theophyllin, Theobromin, Aminophyllin, Hypoxanthin, 1,7-Dimethylxanthin, 3-Isobutyl-1-methylxanthin, 3-Methylxanthin, 3-Ethyl-1-propylxanthin, 3-Allyl-1-ethyl-8-hydroxyxanthin, 3,8-Dimethyl-2-thioxanthin, 1-Ethyl-3-isobutylxanthin, 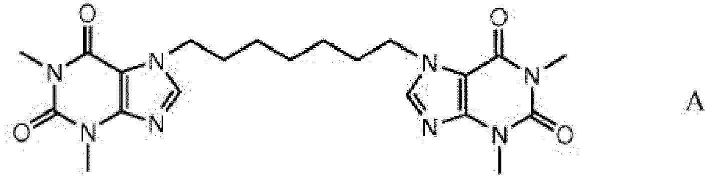 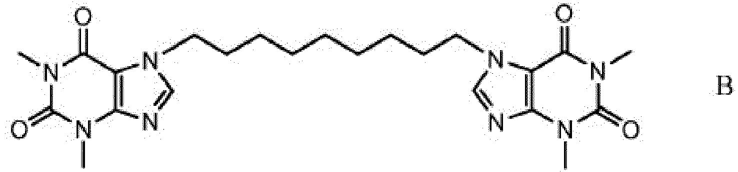 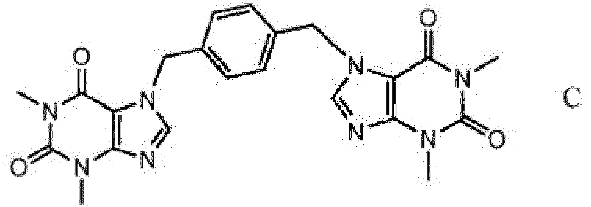 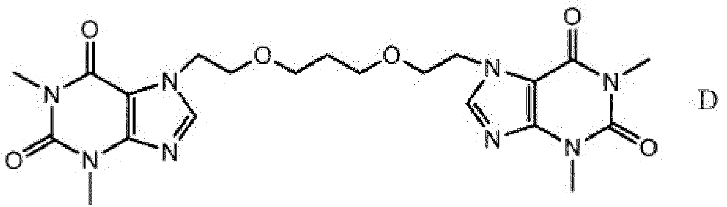 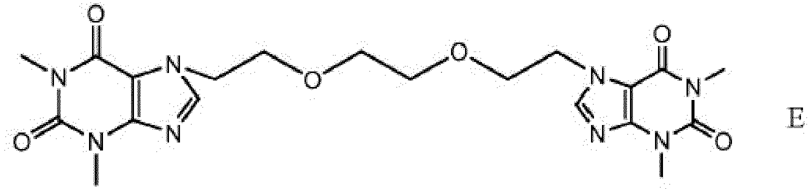 und

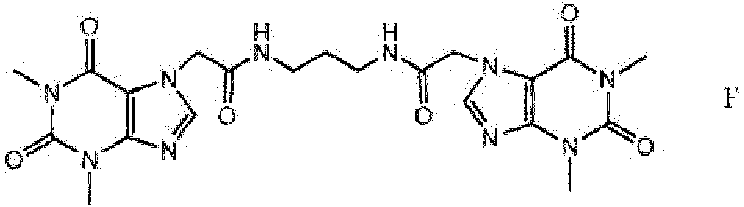

5. Die Verbindung zur Verwendung gemäß Anspruch 4, wobei das Derivat Theophyllin, Theobromin, Aminophyllin, Hypoxantin, 1,7-Dimethylxanthin, 3-Isobutyl-1-methylxanthin, 3-Ethyl-1-propylxanthin oder 3-Allyl-1-ethyl-8-hydroxyxanthin ist.

6. Die Verbindung zur Verwendung gemäß einem der Ansprüche 3 bis 5, wobei die Zusammensetzung als pharmazeutische Zusammensetzung, Nahrung, Nahrungsinhaltsstoff oder -ergänzungsmittel, nutrazeutische Zusammensetzung, Additiv für ein natürliches Produkt formuliert ist oder in dem Extrakt eines natürlichen Produkts vorliegt.

7. Die Verbindung zur Verwendung gemäß einem der Ansprüche 3 bis 6, wobei die Zusammensetzung in der Form eines Feststoffes oder einer Flüssigkeit vorliegt.

8. Die Verbindung zur Verwendung gemäß einem der Ansprüche 3 bis 7, wobei die Zusammensetzung in einem Milchprodukt, einem Getränk oder Cerealien vorliegt.

9. Die Verbindung zur Verwendung gemäß einem der vorhergehenden Ansprüche, die auf oralem, rektalen, nasalen, topischen, vaginalem, parenteralen, transdermalen, intraperitonealen, intrapulmonalen und intranasalen Weg verabreicht wird.

## Revendications

1. Composé qui est la caféine 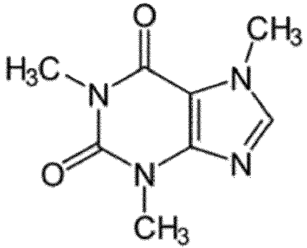 pour une utilisation dans la réduction de la sévérité des symptômes de la dystrophie myotonique de type 1 ou de type 2 chez un sujet souffrant de dystrophie myotonique de type 1 ou de type 2 par augmentation de la quantité de protéine MBNL libre, lesdits symptômes comprenant ceux affectant le coeur, le système nerveux central, le muscle lisse, le système hormonal, le système immunitaire, la vision, le système de reproduction et la peau.

2. Composé pour une utilisation selon la revendication 1, qui est sous la forme d'un tautomère, d'un solvate, d'un hydrate ou d'un sel pharmaceutiquement acceptable de celui-ci.

3. Composé pour une utilisation selon la revendication 1 ou 2, qui est inclus dans une composition.

4. Composé pour une utilisation selon la revendication 3, dans lequel ladite composition comprend en outre au moins une xanthine ou un dérivé de celle-ci, dans lequel ledit dérivé est choisi dans le groupe constitué par la théophylline, la théobromine, l'aminophylline, l'hypoxanthine, la 1,7-diméthylxanthine, la 3-isobutyl-1-méthylxanthine, la 3-méthylxanthine, la 3-éthyl-1-propylxanthine, la 3-allyl-1-éthyl-8-hydroxyxanthine, la 3,8-diméthyl-2-thioxanthine, la 1-éthyl-3-isobutylxanthine, 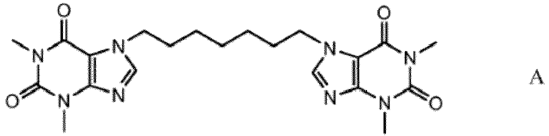 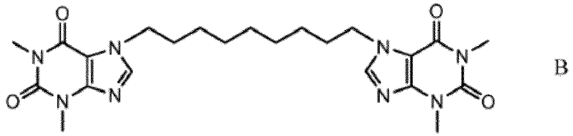 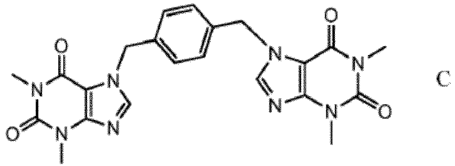 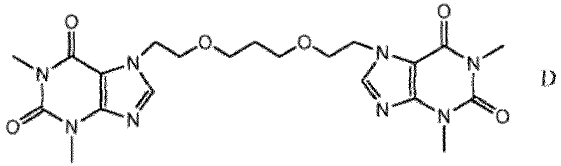 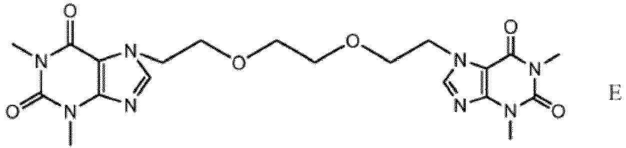 et

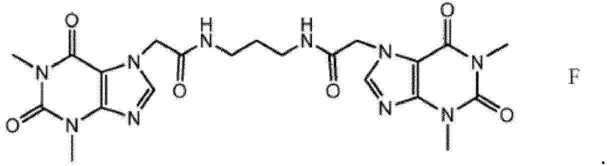

5. Composé pour une utilisation selon la revendication 4, dans lequel ledit dérivé est la théophylline, la théobromine, l'aminophylline, l'hypoxanthine, la 1,7-diméthylxanthine, la 3-isobutyl-1-méthylxanthine, la 3-éthyl-1-propylxanthine ou la 3-allyl-1-éthyl-8-hydroxyxanthine.

6. Composé pour une utilisation selon l'une quelconque des revendications 3 à 5, dans lequel ladite composition est formulée sous forme de composition pharmaceutique, d'aliment, d'ingrédient ou complément alimentaire, de composition nutraceutique, d'additif pour un produit naturel ou est présente dans l'extrait d'un produit naturel.

7. Composé pour une utilisation selon l'une quelconque des revendications 3 à 6, dans lequel ladite composition est sous la forme d'un solide ou d'un liquide.

8. Composé pour une utilisation selon l'une quelconque des revendications 3 à 7, dans lequel ladite composition est présente dans un produit laitier, une boisson ou des céréales.

9. Composé pour une utilisation selon l'une quelconque des revendications précédentes, qui est administré par voie orale, rectale, nasale, topique, vaginale, parentérale, transdermique, intrapéritonéale, intrapulmonaire et intranasale.
